(19) **Europäisches Patentamt / European Patent Office / Office européen des brevets**

(11) **EP 3 734 604 A1**

(12) # EUROPEAN PATENT APPLICATION
published in accordance with Art. 153(4) EPC

(43) Date of publication:
**04.11.2020 Bulletin 2020/45**

(21) Application number: **17936316.3**

(22) Date of filing: **29.12.2017**

(51) Int Cl.:
*G16H 10/60* (2018.01)  *G16H 50/00* (2018.01)

(86) International application number:
**PCT/RU2017/000819**

(87) International publication number:
**WO 2019/132685 (04.07.2019 Gazette 2019/27)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD TN**

(71) Applicant: **Obshchestvo S Ogranichennoj Otvetstvennostyu "Intellodzhik"**
**Moscow, 121205 (RU)**

(72) Inventors:
- **DROKIN, Ivan Sergeevich**
  **St. Petersburg**
  **197022 (RU)**
- **BUKHVALOV, Oleg Leonidovich**
  **Vsevolozhskij r-n Leningradskaya obl.**
  **188678 (RU)**
- **SOROKIN, Sergey Yurievich**
  **Moskovskaya obl., 142114 (RU)**

(74) Representative: **Berggren Oy, Tampere**
**Visiokatu 1**
**33720 Tampere (FI)**

(54) **METHOD AND SYSTEM FOR SUPPORTING MEDICAL DECISION MAKING**

(57)  A method for supporting medical decision making using mathematical models of patients, implemented on a server, includes: generating a training dataset containing electronic medical records of patients grouped by patient; pre-processing the data contained in the medical records of patients selected from the training dataset; converting the processed data into a sequence of medical facts with respect to each patient, using medical ontologies; automatically tagging the resulting sequence of medical facts with respect to each patient, using facts of interest extracted from the patient's medical record; training initial representations independently for each modality; training combined representations; training final models and aggregation parameters; obtaining the medical record of a patient not included in the training dataset; pre-processing the data contained in the patient medical record obtained; converting the pre-processed data into a sequence of medical facts, using medical ontologies; sending the resulting set of facts for input into the models generated; determining a diagnosis, and also conducting an analysis and predicting the most probable disease development with respect to the patient according to the set of facts presented.

EP 3 734 604 A1

**Description**

**Field of the Invention**

This technical solution relates to an artificial intelligence field, namely, to medical decision support systems.

**Background**

Detection and diagnosing of patient diseases is a complex problem which is commonly addressed by doctors. There are many factors which could affect the doctor diagnosis result - doctor's experience, attentiveness, current case complexity. Different decision support systems are used to eliminate these drawbacks.
In a majority of approaches the decision support system enables to detect presence or absence of some pathologies (diseases), however, it cannot make an analysis and prognosis of disease course for a particular patient.

**Summary of the Invention**

This technical solution enables to create a patient mathematical model whereby it possible to increase accuracy of diagnosis and make an analysis and prognosis of disease course for a particular patient.
Method supporting a medical decision using patient representation mathematical models, performed on the server, comprises the following steps:

forming a training dataset comprising electronic health records of patients grouped by each patient;

performing a preliminary processing of data contained in the electronic health records selected from the training dataset;

transforming the processed data into a sequence of medical facts per every patient using medical ontologies;

performing automatic layout of the obtained sequence of medical facts per every patient using diagnoses or other facts of interest extracted from the health records;

performing training of primary representations individually for each of modalities;

performing training of joint representations;

performing training of final models and aggregation parameters;

obtaining a health record of a patient that is not included into the training dataset;

performing the preliminary processing of data contained in the obtained health record of the patient;

transforming the preliminarily processed data into a sequence of medical facts using medical ontologies;

submitting the obtained sequence of medical facts to an input of the final models;

making a diagnosis and also making an analysis and prognosis of a disease course for the patient that correspond to the obtained sequence of medical facts with greatest probability.

The system of medical decision support using patient representation mathematical models comprises at least one processor, random-access memory, storage device comprising instructions downloaded into the random-access memory and executed by at least one processor, the instructions comprise the following steps:

forming a training dataset comprising electronic health records grouped by patients;

preliminary processing of data contained in electronic health records selected from the training dataset;

transforming the processed data into a sequence of medical facts per every patient using medical ontologies;

making automatic layout of the obtained sequence of medical facts per every patient using diagnoses or other facts of interest extracted from the health record;

training of primary representations individually for each of modalities;

training of joint representations;

training of final models and aggregation parameters;

obtaining a health record not included into the training dataset;

preliminary processing of data of the obtained health record;

transforming the preliminarily processed data into a sequence of medical facts using medical ontologies;

submitting the obtained set of facts to the formed models input;

making a diagnosis and also making an analysis and prognosis of a patient disease course with the utmost probability corresponding with the submitted set of facts.

In one of its broad aspects this technical solution enables to model processes and tendencies in a patient organism, define effect of medicines and assigned therapy, define patient mortality after operations or assigned therapy.

**Detailed Description of the Invention**

The below terms and definitions are used in this technical solution.

**Vector Representation** - a common name for different approaches to language modeling and training of representations in natural language processing, aimed at word (and possibly phrases) matching from a dictionary of vectors from $R^n$ for n, of far lesser quantity of words in the dictionary. Distributional semantics is the theoretical foundation for vector representations. There are several methods for creation of such matching, for example, using of neural networks, methods of dimensionality reduction as applied to word co-occurrence matrices and explicit representations trained with word occurrence contexts.

**Patient Vector Representation** - patient mathematical model based on patient physiological data, anamnesis, history of diseases and their treatment (methods and course of treatment, prescribed medicines, etc.), etc., enabling to make a prognosis of disease course, diagnose, make recommendations, treatment strategies, etc. for a particular patient.

**Histology** - a branch of biology dealing with structure, activity and development of living organism tissues.

**Human Histology** - a branch of medicine dealing with structure of human tissues.

**Distributional semantics** - a branch of linguistics dealing with computing of the degree of semantic similarity between linguistic units based on their distribution in large linguistic data sets (text corpora). Distributional semantics is based on distributional hypothesis: linguistic units occurred in similar contexts have close meanings.

**Metadata** - information about the other information, or data related to additional information about contents or object. Metadata disclose information about features and properties characterizing some essentials which enable to search and control them automatically in large information flows.

**Data Modality** - data ownership to some data source defining the said data structure, their format and also enabling to correlate the said structure with any system of organs and/or nosologies and/or procedures. As the patient data collecting means as the patient itself could be a source of data.

**Ontology** - comprehensive and detailed и formalization of some field of knowledge by means of a conceptual scheme. Generally, such scheme consists of hierarchical data structure comprising all relevant classes of objects, their links and rules (theorems, constraints) recognized in this field.

**Regularization** (in statistics, machine learning, inverse problem theory) - method of adding some additional information to a statement with the purpose of solving ill-posed problem or preventing retraining. This information is often given in the form of a penalty for model complexity, for example, it could be constraints of the resulting function smoothness or constraints on vector space norm.

**Stemming** - a process of finding a word stem for the given source word. The word stem is not necessarily the same as the morphological word root.

**Fact** (medical fact) - data describing a patient, including methods of its treatment and linking of the said data with the other medical facts.

**Electronic health record** (electronic medical record - EMR) - database comprising patient data: patient physiological data, anamnesis, history of diseases and their treatment (methods and course of treatment, prescribed medicines, etc.), etc. Also the electronic health record comprises patient records including at least the following data: record adding date, codes of diagnoses, symptoms, procedures and medicines, text description of case history in natural language, biomedical images associated with the case history, results of patient study and analyses.

Medical personnel or other user of the medical information system downloads the patient data comprising health record, patient physiological data and other information to the server through this system interface.

In the other embodiment the data could be downloaded to the server automatically, without human involvement, for example, at analysis collection and analysis, treatment procedures, etc.

During patient visiting a doctor, undergoing an examination, delivering medical tests or during other medical procedures the data on each such procedure are formed (filled in and stored) in the medical information system. The data may include, but not limited to, patient examination records, codes of diagnoses, symptoms, procedures and medicines prescribed and/or taken by a patient, description of case history in natural language, biomedical images, results of analyses and studies, observation results and results of physiological data measurements, ECG, EEG, MRT, ultrasound investigation, biopsy, cytologic screening, X-ray, mammography.

The said data can be represented in, but not limited to, text, table format, in the form of time-series, images, video, genomic data, signals. The data can also be presented in structured and unstructured form. Additionally, links between the above data could serve as the data.

Analyses may include, but not limited to, analysis of blood, cerebrospinal fluid, urine, feces, genetic tests, etc. Within the scope of the technical solution there are no restrictions to analysis types.

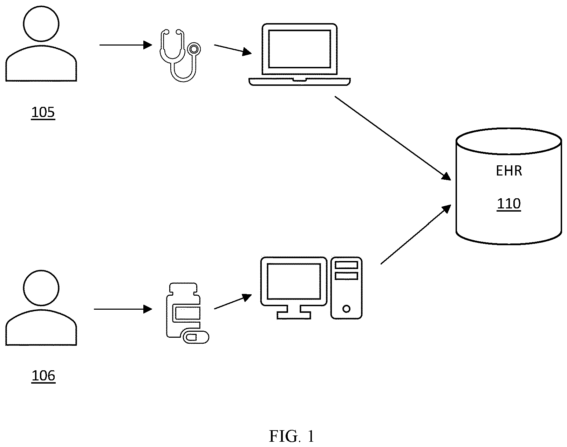

Let us consider an example illustrated in **Fig. 1:**

One patient **105** visits a specialist doctor for initial evaluation. The doctor carries out necessary medical activities, then forms a description of the patient's symptoms, gives a prescription for tests. Then the doctor inputs this information into a computer through the interface of the medical information system **110,** after that these data are stored in the electronic health record. The other patient **106** revisits a therapeutist. The therapeutist prescribes medicines to the patient and inputs these data to the electronic health record. Through this process the required record set is formed in electronic health records per each patient, and this set can be used by other doctors or decision support systems.

In order to create medical decision support systems, it is required to collect definite data amount enabling to train the system to recognize diagnoses, groups of diagnoses or facts according to available medical data. When the required data amount is collected in the medical information system, it can be used as a training dataset. The majority of the existing decision support systems use machine learning in its different manifestations.

One of the important components in the medical decision support system is the patient vector representation (patient mathematical model) enabling to make a prognosis of disease course, diagnose, make recommendations, treatment strategies, etc. for a particular patient.

In one of the embodiments the functional enabling to form the patient vector representation is located in the separate server.

In one of the embodiments the functional enabling to form the patient vector representation is located in the same server where the medical decision support system is located.

In one of the embodiments the functional enabling to form the patient vector representation is a cloud service using cloud computing in the distributed server system.

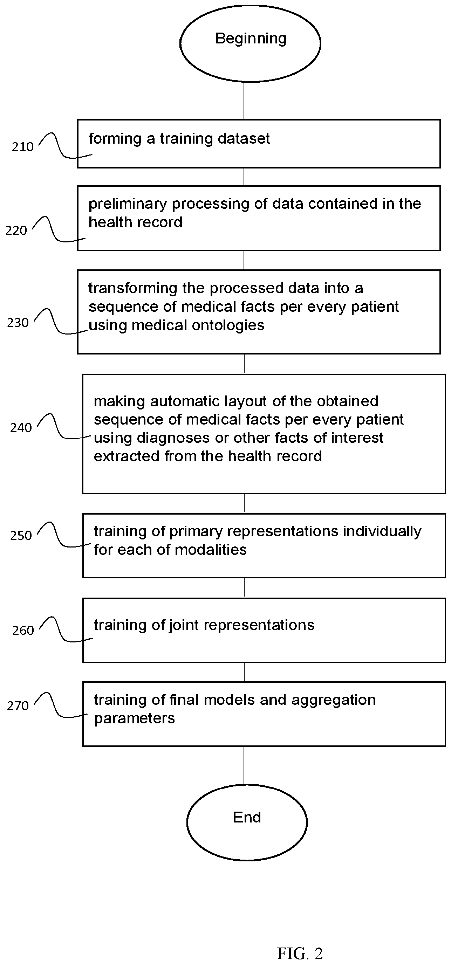

At the first step, in order to form the patient vector representation, the training dataset **210, Fig. 2** is formed, which will be subsequently used for algorithm machine learning, including deep learning algorithms).

In one of the embodiments the training dataset is formed by a user of the medical information system, by selection of patient records.

In one of the embodiments the selection of records can be performed according to the specified criteria. At least the following can serve as criteria:

   • rules of including to/excluding from the training dataset:

      ◦ patients with anamnesis from the specified set of anamneses (e.g. the patients with oncologic anamnesis only);
      ◦ patients complying with the specified gender or age parameters (e.g. men aged from 30 to 45 years only);
      ◦ patients related to the patients already included into the training dataset, and the relation is defined at least by similarity of anamneses, treatment methods, etc.

   • rules of including the training datasets previously formed.

Within the scope of this technical solution the training dataset comprises the electronic health records grouped by patients. The electronic health record used for the training dataset formation comprises patient records including at least the following data: record adding date, codes of diagnoses, symptoms, procedures and medicines, text description of case history in natural language, biomedical images associated with the case history, results of patient study and analyses.

Below is a citation of a fragment from a health record as an illustrative example:

01.01.2001
The patient complained about fever jump and wet cough. The patient was sent to chest x-ray and took an antifebrile <ANTIFEBRILE> in a dose of <DOSE>.

02.01.2001
Pneumonia diagnosis is confirmed. The following therapy is assigned <THERAPY DESCRIPTION IN NATURAL LANGUAGE>

The used data presentation formats could vary and change depending on the technologies applied. The described formats are not the only possible and are described for the best understanding the concepts of this technical solution.

Electronic health record can be presented in the formats: openEHR, HL7, etc. Selection of format and standard does not affect the essence of the technical solution.

In one of the embodiments the health record is a set of fields comprising at least parameters describing:

- patient state;
- methods of patient treatment (techniques, methods of their application, characteristics);
- means used for patient treatment (medicines, doses, etc.);
- analyses results, etc.;

and metadata linking the described parameters with the parameters from the other records.

If the records in the training dataset are not grouped per patient, they are grouped after data acquisition using the known algorithms or functions (e.g. any sorting and sampling known from the prior art, including use of GROUP BY, ORDER BY commands in SQL queries at data sampling from the databases).

Health record data can be represented in, but not limited to, text, table format, in the form of time-series, images, video, genomic data, signals. The data can also be presented in structured and unstructured form.

Record adding date can store only the date, date and time, time stamp, and the said records can comprise the said time objects as in the absolute form as in the relative form (relative to the time objects from the other records).

Codes of diagnoses, symptoms, procedures and medicines can be presented in MKB format (e.g. MKB-10), SNOMED-CT, CCS (Clinical Classifications Software), etc. Selection of format does not affect the essence of the technical solution.

The analyses results can be presented in a table form.

Text description of the case history in structured and unstructured form (description in natural language).

Biomedical images can be presented in the form of image (jpg, png, tiff and other graphic formats), video (avi, mpg, mov, mkv and other video-formats), 3D photo, 3D video, 3D models (obj, max, dwg, etc.). Results of ECG, EEG, MRT, ultrasound investigation, biopsy, cytologic screening, X-ray, mammography, etc. can be presented in the form of biomedical images.

RNA sequencing data can be presented in TDF format (tiled data format), unindexed formats such as GFF, BED and WIG, indexed formats such as BAM and Goby, and also in bigWig and bigBed formats.

The above formats indicate what minimum software is designed for operation with the above data (creation, modification, etc.).

When the training dataset is formed and received at the server, the server executes preliminary processing of the data **220,** contained in the health records of the patients, selected from the training dataset.

Data preliminary processing is domain-specific and depends on data type and data source.

Special data handlers are assigned for each data type and/or data source. If no handler is not assigned or not required for a data type and/or data source, empty handler is used or the handler is ignored for such data type.

In one of the embodiments the data type is defined on the basis of metadata specified at least for one type of data field in the electronic health record.

For example, in dicom, the metadata indicate data modality in an explicit form and the data modality is interpreted according to the dicom internal definition.

In one of the embodiments the data type can be defined by means of signatures. Provided that, the server or external source has a database of signatures by means of which the data type is defined in the record.

For example, availability of GIF89a sequence of bytes beginning of data (field or file) means that it is a bitmap image in GIF format, and availability of BM bytes means that it is a bitmap image in BMP format.

In one of the embodiments the data type can be defined on the basis of the record information using the preliminarily specified rules.

For example, type of image data (Bitmap, Icon), multimedia data (video, sound), stored in the executable file resources (PE-file, .exe) is defined on the basis of the said executable file .rdata structure analysis.

In one of the embodiments the one type data can be converted into the other type data (video - into a set of images, and vice versa, 3D object - into the said object mapping image, and vice versa, etc.).

For example, for CT images there could be assigned a handler transforming them into a series of bitmap images with possible normalization, if the parameters of the device used to take this image are known.

For the other example, for a text there could be assigned a handler which executes standard text NLP transformations (generally, it is lower casing, number supersedence, deleting stop words and prepositions, stemming).

For the other example, for a text in natural language there could be assigned a handler which forms a sequence of medical facts from the text by its mapping to the terms of medical ontology and/or dictionary of medical terms.

In one of the embodiments for analysis of the text in natural language there could be used algorithms of at least lexical analysis and syntactic analysis, known from the prior art, based on which the lexical units are extracted from the text and combined into the objects representing a sequence of medical facts.

In one of the embodiments when mapping the text each medical fact is annotated (marked) with date and/or time corresponding with the date and/or time of the current record from the health record.

For example, if the handler handles a field containing a text in natural language from the patient record dated 20.01.2017, all medical facts will be annotated (marked) with the date 20.01.2017.

In one of the embodiments for analysis of the text in natural language there could be used algorithms of at least lexical analysis and syntactic analysis, known from the prior art, based on which the lexical units are extracted from the text and combined into the objects representing a sequence of medical facts.

In the other embodiment for analysis of the text a preliminarily trained (by one of the machine learning methods) model of text recognition is used, and a set of medical facts is formed as result of this model operation. Moreover, the said model can be retrained (using supervised learning methods), if the formed medical facts do not comply with the criteria specified before (for example, when results are analyzed by a specialist).

In one of the embodiments of the handler for natural language the handler searches for each word (after preprocessing) from the text in an ontology or dictionary. If a word is found in an ontology or dictionary, the handler saves the ontology concept or a word from the dictionary corresponding to it, and the words not found in the ontology or dictionary are rejected.

In one of the embodiments more complicated rules (procedures) of text mapping in natural language into a sequence of facts could be used.

For example, for some concepts there could be used additional templates (regular expressions) enabling to extract related concepts and/or values.

In one of the embodiments ontologies and/or dictionaries are localized in the server.

In the other embodiment the server can receive ontologies and dictionaries from external sources.

For example, through the Ontology Lookup Service, which provides web service interface for query of many ontologies from one place with a unified data output format.

In one of the embodiments any source of medical data, from which a knowledge forest (a variety of acyclic oriented knowledge graphs) can be formed, could be used as a knowledge source instead of ontologies and dictionaries. Medical schemes-guidelines, in particular, etc. belong to such sources.

In one of the embodiments specialized medical articles and/or training manuals could be used as a knowledge source. Provided that, the preliminarily found articles are processed by text recognition methods, known from the prior art (by means of the above mentioned lexical and syntactic analyses, using trained text recognition models, etc.).

In the other embodiment Open Biomedical Ontologies are used as a knowledge source.

In one of the embodiments the handler normalizes the data (individual normalization rules could be used for every data type).

For example, the handler can normalize some specific blood measurement values (feature scaling) in the table form, and parameters of such transformation are computed at the training dataset. In particular, sample mean a and variance $\sigma^2$ are computed, and

$$\tilde{x} = \frac{x - a}{\sigma}$$

In the other example for an image, where each pixel value corresponds to the value of the measured medium density according to Hounsfield intensity, the handler can execute mapping of the Hounsfield scale into [-1, 1] range (normalize all whole-number values within [0..255] range for black and white images to actual values within [-1.0..1.0] range). In particular, normalization can be described by the formula:

$$x' = \frac{x - x_{min}}{x_{max} - x_{min}}$$

$$y = y_{min} + (y_{max} - y_{min}) \times x'$$

where

$x$ - normalizable value in the value space $\{X\}$;
$x_{min}$ - minimum value in the value space $\{X\}$;
$x_{max}$ - maximum value in the value space $\{X\}$;
$x'$ - normalized value in the value space $\{X\}$;
$y_{min}$ - minimum value in the value space $\{Y\}$;
$y_{max}$ - maximum value in the value space $\{Y\}$;
$y'$ - normalized value in the value space $\{Y\}$;

In the other example the data from the table containing specific blood measurements are subjected to preprocessing - data normalization (reducing each of the parameters to zero mean unit variance, parameters of such transformation are computed at the training dataset).

In the other example the data provided in the form of RGB images obtained from a microscope, are subjected to postprocessing - from probability to class binarization. If from the perspective of the model the pathology probability exceeds the specified threshold, the image is marked as containing a pathology otherwise than not containing.

In one of the embodiments of the handler filters noise or reduces noise of the data being analyzed (process of noise elimination from the useful signal to improve its subjective quality or to reduce the error level in digital data transmission channels and storage systems). In particular, at image processing there could be used one of the spatial noise suppression methods (adaptive filtering, median filtering, mathematical morphology, discrete wavelet-based methods, etc.) for video - one of the methods of temporal, spatial or spatio-temporal noise suppression.

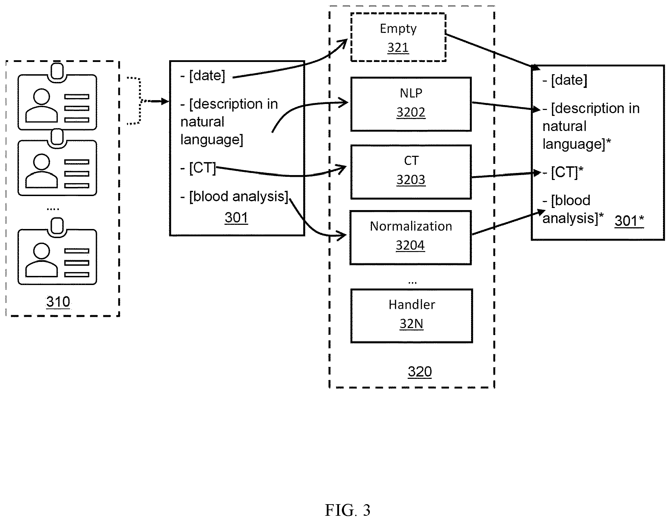

Let us consider an example illustrated in **Fig. 3**:

Let us assume that there is the training dataset **310,** consisting of patient records. The server executes preprocessing of the data selected from the record fields for each sample record. For example, from the training dataset **310** the server extracts one record **301** from the patient health record, defines field contents and/or types of data contained in the record. In the example given the record **301** comprises date, description in natural language, CT images, blood analysis. Then the server processes the data for each record field by means of the corresponding handler from the handler pool **320** (the handlers **3201..32N**). For example, date can be processed by the empty handler **3201,** text in natural language - by the handler **3202** executing standard text NLP processing, CT images - by CT handler **3203,** blood analysis - by the handler**3204** normalizing the data. After processing the patient record **301** comprises the processed data **301*,** where '*' symbol next to a field means that this filed comprises the changed records (different from the original ones).

In one of the embodiments the handler is formed as using one of the scripting languages, as in the form of plug-ins, libraries (including, executable PE-files, e.g. dll).

In one of the embodiments the server has a built-in set of procedures for primitive actions on data types. Combination of these procedures in the order appropriate for the User enables to create the handlers with no outside help. In this case the handlers are created by means of built-in support of scripting languages or through interface platform enabling to create such handlers.

When the server has executed the required data preprocessing, the server maps **230** the processed data into a sequence of medical facts per every patient using medical ontologies.

The whole health record is mapped by the server into a sequence of medical facts about the patient. The facts may contain additional information, e.g. biomedical image, ECG, analysis results, etc.

Let us consider two records from the electronic health record and the result of their transformation into a sequence of medical terms as an illustrative example:

Record before transformation:

```
01.01.2001
The patient complained about fever jump and wet cough. The patient was sent to chest x-ray and
took an antifebrile <ANTIFEBRILE> in a dose of <DOSE>.
01.01.2001
<Chest x-ray image>

02.01.2001
Pneumonia diagnosis is confirmed. The following therapy is assigned <THERAPY
DESCRIPTION IN NATURAL LANGUAGE>
Medical facts after transformation.
01.01.2001
<FEVER>
<COUGH>
<CHEST X-RAY. Image>
<ANTIFEBRILE, DOSE>

02.01.2001
<PNEUMONIA>
<MEDICINE 1, DOSE>
<MEDICINE 2, DOSE>
<PROCEDURE 1>
```

After health record transformation into a set of medical facts the server makes automatic layout of the obtained sequence of medical facts **240** per every patient using diagnoses or other facts of interest extracted from the health record. If the data are laid out, the server skips this step.

In one of the embodiments the facts of interest are specified by the server user or accumulated in the users of this technical solution (e.g. doctor).

For example, lists of inclusive and exclusive criteria for clinical tests, i.e. lists of criteria which a person shall comply with in order to be included into the clinic (inclusive lists) or otherwise, be excluded from the clinic or not to be admitted to the clinic (exclusive lists).

In the other example the liver cancer with maximum 5 mm tumor could be an inclusive criterion.

In the other example smoking or patient age over 65 could be an exclusive criterion.

In one of the embodiments the facts of interest are extracted from the external sources.

For example, the facts of interest can be extracted from medical information systems.

Then the server arranges and groups the facts by time-wise examinations. Such grouping is necessary to consider a group of facts within one examination simultaneously.

In one of the embodiments the analyses could relate to the attendance when they were ordered, or they are separated into individual essence (individual examination).

In one of the embodiments CT, MRT and histology relate to an individual examination.

In one of the embodiments at least all examination methods, containing unprocessed data (not doctor's report but the immediate result in the form of image, video, time marks) relate to individual examinations. If only a doctor's report or the mere fact of examination is available, such data are considered as a part of examination.

For such grouping by examinations the server uses information about time and/or date related to each fact.

Then the server forms pairs {set of facts, diagnosis} or {set of facts, fact of interest} based on grouping by examinations.

In one of the embodiments the pairs are formed by simple searching.

Then the server prepares the training dataset for each data modality. As mentioned earlier, data of histology, X-ray, CT, mammography, etc. can serve as data modality.

For example, for the purpose of forming the training dataset for CT modality the server selects records comprising CT:

```
{<CT, image>, <DIAGNOSIS_i>}, {<CT, image>, <DIAGNOSIS_j>},
{<CT, image>, <DIAGNOSIS_k>}, ...
For example, for the concepts (terms) the server selects the following records:
{ [<FEVER>], <PNEUMONIA>}
{ [<FEVER>, <COUGH>], <PNEUMONIA>}
{[<FEVER>, <COUGH>, <CHEST X-RAY>], <PNEUMONIA>}
{[<FEVER>, <COUGH>, <CHEST X-RAY>, <ANTIFEBRILE>], <PNEUMONIA>}
```

Then, after forming the training datasets for each modality the server executes training of primary representations **250** individually for each of the modalities.

A model (group of models) is set at the server for each of the modalities for forecasting training of the diagnoses revealed in this training dataset and available in these modalities.

In one of the embodiments the following machine learning algorithms can serve as a model:

- linear regression;
- logistic regression;
- nearest-neighbor k algorithm;
- random forest;
- gradient boosting on trees;
- Bayesian classifiers;
- deep neural networks (fully connected, convolution, recurrent, their combinations).

In one of the embodiments the said model complies with the requirement - correspondence to the modality with which this model will work.

For example, convolution networks are used for images.

In one of the embodiments several models are specified for each modality.

In one of the embodiments the modalities are grouped into clusters (e.g. all histology, X-ray, mammography, etc.) with similar model architecture (the same n-parameter family) and trainable together, and each cluster model has different weight sets.

In one of the embodiments a set of model n-parameter families is formed for each modality. N-parameter family means that there is a common type of models with some set of parameters, which defining specifies the model unambiguously.

For example, if a neural network is used as a model, one of the examples of the n-parameter family is a multilayer perceptron, and number of layers and number of neurons in the layers will be the parameters. The other example - any neural network with fixed architecture, which generates a n-parameter family (e.g. family intended for image segmentation, for image classification, etc.).

Within the scope of this technical solution it is supposed to use the following main n-parameter families:

- convolution neural networks for working with images, video, signals;
- recurrent neural networks for working with sequences of facts in the patient health record and for building forecast models, for processing unstructured text information;
- Bayesian approach and decision trees for working with table data.

Let us take a closer look at the example of working with images.

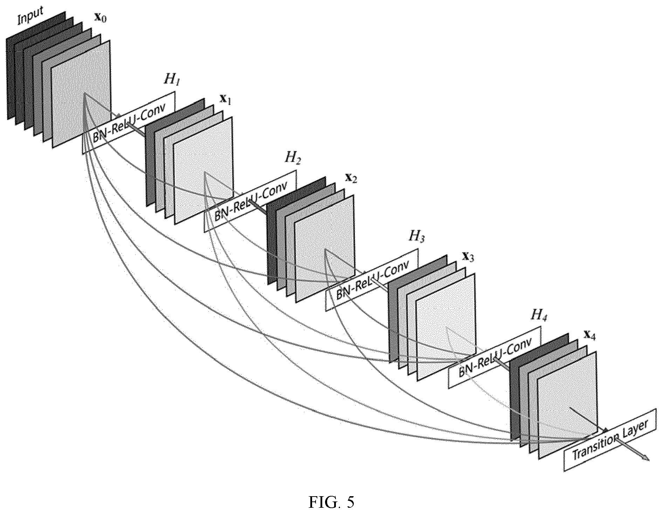

There are the following main problems in the working with images: image classification (assign one or several classes or marks to each image), image segmentation (assign one or several marks to each image pixel), localization of objects of interest (build an enclosing rectangle inside which there is an object of interest, for each object in the image). An architecture solving the problem is assigned for each of these problems. Generally, the modalities differ in size of input image and in number of target marks/objects of interest. Dense-net is taken as a basis of each such model, which architecture concept is presented in Fig. 5.

The concept of this architecture is using the additional ways for information movement within the model, that enables to train effectively even very large models with a large number of convolution layers. At assigned modality, size of input image and number of classes such model forms n-parameter family, and the neural network weights are just the parameters of the family. They are defined in the process of training, during which images and target marks are presented to the model, and the neural network changes its weights so that its response matches the content in the training dataset layout (so called target values or target response).

Then, for each modality the server searches the family parameters giving optimal result in this training dataset.

In one of the embodiments at least the following is used for searching the family parameters giving optimal result:

- Monte-Carlo method;
- Bayesian optimization.

In one of the embodiments cross validation is used for model evaluation, on the basis of which the best parameter model is selected for this training dataset for this modality. Cross validation is executed as follows. Dataset $X^L$ is partitioned $N$ by different methods into two disjoint subsets:

$$X^L = X_n^m \cup X_n^k,$$

where

$X_n^m$ - training subset of $m$ length,

$X_n^k$ - validation subset of $k = L - m$ length,

$n = 1.. N$ - partition number.

For each partition $n$ the algorithm is built

$$a_n = \mu(X_n^m)$$

and quality functional value is computed

$$Q_n = Q(a_n, X_n^k)$$

The arithmetic mean $Q_n$ for all partitions is called *cross validation evaluation:*

$$CV(\mu, X^L) = \frac{1}{N} \sum_{n=1}^{N} Q(\mu(X_n^m), X_n^k).$$

It is the cross-validation evaluation that is used for selection of the best model.

In one of the embodiments the models are added as data are collected and new patient data sources are added.

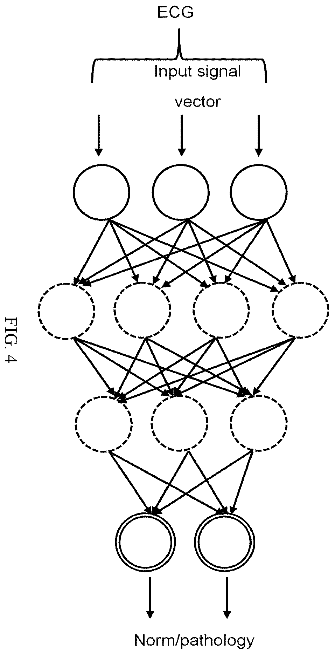

For example, new type of examination (e.g. ECG) has become available in the medical information system. Then, the training dataset is formed for ECG modality, then neural network (Fig. 4) model (group of models) is trained at this training dataset, from which the representation of this modality is obtained.

After the models have been trained, the server forms primary vector representations for each modality. For this purpose, the server sends preprocessed patient data to input of each model trained for this modality, defines model output values and weight values of the last hidden layer of this model for each record. Weight values of the last hidden layer further will be used by the server as primary vector representations, which are modality mapping into the vector of fixed sized defined by the model. As a result, the server forms new set of data, which is a transformation of the original one.

Each modality has its own vector dimension, e.g. if the modality - $m_u$, and the generic space dimension - $n$, the mapping is built:

$$R^{m_u} \rightarrow R^n$$

For example, suppose

$$x \in R^{m_u}, y \in R^n,$$

then, the mapping:

$$y = Ax + b,$$

where

$$A \in R^{(n,m_u)}, b \in R^n$$

In the other example

$$y = f(Ax + b),$$

where

    $f$ - nonlinear function (e.g. ReLU, sigmoid, etc.).
    I.e. A is a matrix of size $n, m_u$, and $b$ is a vector of size $n$

In one of the embodiments the text vector representation is built in the space where the primary vector representations of all the other modalities are mapped to, i.e. primary vector representation of the text modality is mapped to the space of common representations by identity mapping.

If not, a neural network model is used there are two possible scenarios:

- model output as such is taken as data representation;
- neural network model is a classifier mapping input data into a set of facts for which there are already vector representations. Since it is guaranteed that any model will generate a probability vector of fact availability, the vector representation will be probability weighted by amount of fact vector representations.

For example, the following model is built:

$$f(x, \xi): X \rightarrow Y,$$

where

    X - set of features,
    $Y = \{y_i, i = 1, ... , n\}$ - set of target facts
    S - model parameters.

Without loss of generality the problem can be reformulated as follows:

$$P = f(x, \xi): X \rightarrow R^n,$$

and

$$P = (p_1, p_2, ..., p_n); \sum_{i=1}^{n} p_i = 1, p_i \geq 0.$$

With such restrictions to the model $p_i$ could be interpreted as a probability of fact availability $y_i$ with a patient (or other variants depending on the problem solved, e.g. appearance of the fact with a year horizon, etc).

Having the training dataset $\{x_j, t_j\}, j = 1,..., N$ it is possible to define model parameters $\xi$. Let us call the model parameters obtained during training as $\hat{\xi}$. Also, since each of $y_i$ is a medical fact, the vector representation $V_i$. corresponds to it

Then for a new case we obtain the following: build an input feature vector $\bar{x}$ corresponding to this case; obtain the probability vector $\bar{p} = f(\bar{x}, \hat{\xi})$ corresponding to it; in this case the vector representation of this modality will be built as follows:

$$\bar{V} = \sum_{i=1}^{n} \bar{p}_i V_i.$$

The server forms primary vector representations for each of the modalities that results in a set of vector representations for medical facts and terms (diagnoses, symptoms, procedures and medicines) and the models for mapping the primary vector representations to the joint representations space.

In one of the embodiments the server additionally pretrains vector representations of the medical terms (concepts), for example,

- given an additional data source in the form of large corpus of medical literature;
- given an alternative training corpus which was assembled irrespective of the current one.

The server pretrains medical terms (concepts) using distributional semantics and word vector representation.

Individual context vector is assigned to each word. Set of vectors forms verbal vector space.

In one of the embodiments pretraining of medical terms (concepts) is executed using software tool for analyzing natural languages semantics Word2vec using an ontology for regularization.

Regularization in statistics, machine learning, inverse problem theory - method of adding some additional information to a statement with the purpose of solving ill-posed problem or preventing retraining. This information is often given in the form of a penalty for model complexity.

For example, it could be:

- constraints of the resulting function smoothness;
- constraints on vector space norm;
- regularization on weights and on firing of neurons;
- regularization methods known from the prior art.

This technical solution uses main and common machine learning and deep learning regularization methods known from the prior art. Let us assume that $E$ - error function, minimized during training, $W$ - model weights, A - firing of all neurons of hidden layers (in respect to neural network). Then, one of the most abundantly used regularization techniques named $L_1(L_2)$ regularization can be described as follows: Instead of minimization $E$ the minimization problem is solved

$$E + \alpha L_1(W) \underset{W}{\to} min \ (L_1 \text{ weight regularization}),$$

$$E + \alpha L_2(W) \underset{W}{\to} min \ (L_2 \text{ weight regularization}),$$

$$E + \alpha L_1(A) \underset{W}{\to} min \ (L_1 \text{ firing regularization}),$$

$$E + \alpha L_2(A) \underset{W}{\to} min \ (L_2 \text{ firing regularization}),$$

where

$$L_p(x) = (\textstyle\sum_{i=1}^{n} |x|^p)^{\frac{1}{p}} - L_p\text{- norm}.$$

Different variants of the given cases are possible. Given regularizing summands (terms) place additional (mild) restrictions. I.e. restrictions on the possible model weights not assigned in the form of explicit set of equations and/or inequations generating the set $\tilde{W} \subset R^n$ of valid model weights), that enables to avoid retraining. Also, along with $L_1/L_2$ regularization the following can be used:

- early stop:
  The principle of this method is to select a small testing set from the training dataset, which is not involved into the training explicitly, but it is used for model error measuring during the training process. As soon as the error in this testing set starts to increase, the training stops.

- data augmentation:
  The principle of this method is that with a certain probability the transformation is applied to each example of the training dataset. This transformation does not change the required response or enables to obtain new required response, which will be correct, by applying similar transformation. For example, when classifying chest X-ray image for presence or absence of pneumonia signs, it is possible to apply mirror mapping to input image about the axis, since it obviously will not change the target mark.

- the restrictions could be explicitly imposed on the model parameters through a restriction to the norm value of the model weight vector: $L_1(W) < \gamma$ or $L_2 (W) < \gamma$.

- other regularization methods widely accepted in machine and deep learning can also be applied.

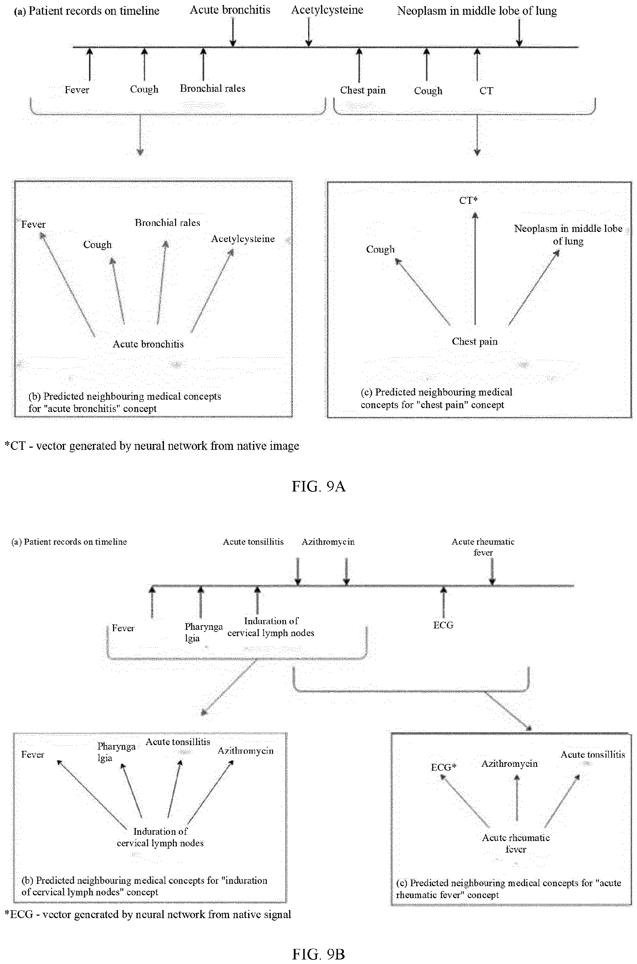

Let us give an illustrative depiction of word2vec tool operation: word2vec input receives a large text corpus and associates a vector with each word outputting word coordinates. First, it creates a dictionary, "learning" from input text data, and then computes word vector representation. Vector representation is based on context similarity: words occurring in the text close to the same words (and, consequently, having the similar sense) will have close coordinates of the vectors-words in the vector representation. Obtained vectors-words (e.g. Fig. 9A for bronchitis and Fig. 9B for rheumatism) can be used for natural language processing and machine learning.

There are two main learning algorithms in word2vec: CBOW (Continuous Bag of Words) and Skip-gram. CBOW - model architecture, which predicts the current word based on the ambient context. Skip-gram architecture functions in a different way: it uses the current word to predict the ambient words. The context word order does not affect the result in any of these algorithms.

The coordinate representations of the vector-words obtained at the output enable to compute "semantic distance" between the words. Based on context similarity of these words the word2vec technique gives its predictions.

In one of the embodiments when using an ontology as regularization (constrains on the space structure) the attention is used.

In one of the embodiments, when using an ontology for regularization (constrains on the space structure), the multilevel (hierarchical) error function, based on relations between the ontology terms, is used. In the particular case the ontology is used in the form of knowledge graph, which specifies the hierarchy of terms and their categories. It enables to arrange the vector representation space beforehand, since it is obvious that similarity in the knowledge graph should mean similarity in the vector space between terms and categories. Using this it is possible to impose a penalty for vector representation training. Thereafter, the penalty is minimized together with the main error function. Let us call the current term vector asc. Binary similarity measure between two terms $c_1$ and $c_2$ against ontology we call as q. If $q(c_1, c_2) = 0$, the terms could be considered similar, if $q(c_1, c_2) = 1$, - remote. Then the ontology error function can be defined as follows:

$$OD(c_i) = \sum_{c_j: q(c_i,c_j)=0} \|c_i - c_j\| - \sum_{c_j: q(c_i,c_j)=1} \|c_i - c_j\|$$

Now, during vector representation training $OD(c)$ could be used in a similar manner to $L_1/L_2$ regularization.

Using of regularization by means of ontology enables to improve model quality without extension of the training dataset. Due to reasonable restriction to the representation space imposed by the regularization the model quality is improved, that enables, in particular, to avoid retraining, make the algorithm more stable relative to overrunning and errors in the training set. Standard methods of classic regularization also impose restrictions on the representation space; however, they only narrow the variants as opposed to the ontology-based regularization, which imposes restrictions on the representation space, based on external information about domain area.

In one of the embodiments the ontology used for regularization is the external parameter relative to the system, which can be specified beforehand and can depend on the corresponding disease code system (e.g. Idc9/10, MKB-10, etc.).

For each neural network model, obtained at this step, the primary vector representation is extracted as the hidden layer output. The said model enables to map input data of the specified modality into the primary vector representation. It requires simple manipulations with the trained model, actually reducing to removing the output layer from the model.

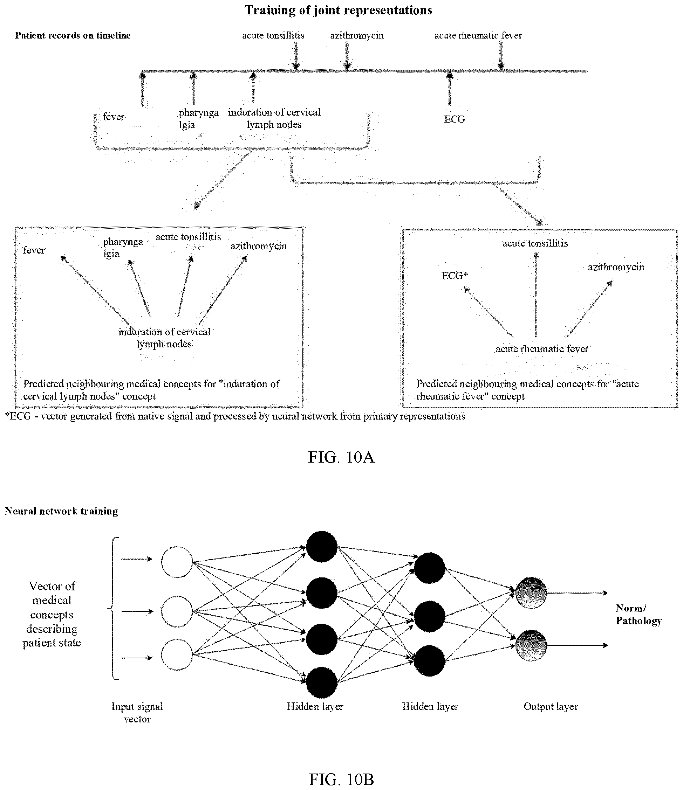

After training and obtaining primary representations the server executes coordinated multimodal machine learning of joint representations **260** (illustrated in Fig. 10) (read more: "Multimodal Machine Learning: A Survey and Taxonomy", Tadas Baltrusaitis, Chaitanya Ahuja, and Louis-Philippe Morency).

In order to use the non-text data in the process of the coordinated multimodal machine learning of joint representations, e.g. medical images, it is necessary to train the model of mapping from this modality space into the generic vector space. For this purpose the primary vectorization of modality and the trainable function of presentation from the primary vector representation int the common one are used, provided that, the above mentioned model (model trained for mapping from the specified modality space into the generic vector space) can be used as the trainable function.

For example, if the model deals with image classification only, then several hidden fully connected layers can follow the last convolution layer. In this case the output of just the last hidden convolution layer but not fully connected layer is taken.

If in the health record non text modality occurs, e.g. CT scan, its primary vector representation is taken, processed using multilayer perceptron, and this perceptron output is considered to be the vector-representation of this image and cosine distance between its neighbors is computed on it.

Thereafter, skip-gram is used, however, when it comes to non-text modalities (e.g. medical images), it is the function output for this modality that is used as their vector representations, provided that, corpus of sequences of medical facts extracted from health records or medical texts is transmitted to skip-gram input.

Then after the coordinated multimodal machine learning of joint representations the server executes learning of final models and aggregation parameters **270.**

Aggregation is obtaining a single vector from the set of vectors where each vector is a medical fact from the health record of the selected patient.

Weight obtained during learning is assigned to each fact. There is formed a set of weights which are used during the process of prognosis/diagnosis for a particular patient - aggregation parameters. Then, the weighted amount is defined - by multiplying each vector in the health record by corresponding weight, and the obtained vectors are summed up. Generally, during aggregation of vector-representations the direct sum of vectors is used $c_{ag} = \sum_{i=1}^{k} c_i,$ where $c_{ag}$ aggregated patient representation, $c_i$ - vector representations of facts in this patient health record. However, this variant of aggregation cannot be always optimal, since each of the facts can have different weight from the perspective of taking a decision for each of the nosologies or for this patient. Therefore, it is suggested to use the following approach as aggregation: $c_{ag} = \sum_{i=1}^{k} a_i c_i,$ $c_{ag}$ where $a_i$ is scalar, $\sum_{i=1}^{k} a_i = 1.$ Each their $a_i$ could be as model explicit parameter and be defined during training, as be the function $a_i = f(i, c_1, c_2, ..., c_k, \psi)$, where $\psi$ are the parameters of this function, which are defined during training together with the other model weights.

Computation graph is built, where the weights are as parameters. Then, the graph parameters are optimized in accordance with the current set of data by gradient descent method. The resulting set of weights is trainable, i.e. it is modified together with the other model weights during training. The weights define the specific function from the n-parameter family, which forms one output vector from several input vectors.

All the aforesaid can be summarized as follows: Classifier training for a group of diagnoses is executed on the basis of graphs, the training dataset is generated from the available EHR automatically based on NLP-techniques (facts extraction + their arrangement in temporal order, then the pairs "facts" - "diagnosis" are generated from them). Selection of classifier is determined by the possibility of working with uncategorized vector features, and in this method, these are multilayer fully connected neural networks with remainders.

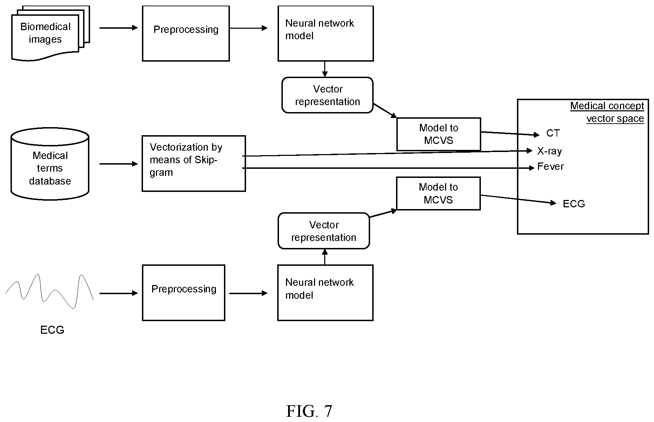

Two pipelines for ECG and biomedical images, e.g. chest CT, are illustrated in Fig. 7 First the data enter the preprocess block. The preprocess block is domain-specific and transforms input data into the sort of data which the model is expected to receive. The transformed data are transmitted to the model corresponding to the said data modality - for example, chest CT is transmitted to the neural network, which analyzes this examination. The model can output the results of two kinds (two outputs): it is the desired model output as such (pathology probability, segmentation maps, etc.) and vector representation of this particular example. The desired model output is transmitted to the postprocess module which is connected to the model, and this block output is demonstrated to a human expert, for example, or sent to a client in the form of a report or in any other form acceptable for it.

The central scheme depicts a vector space of medical concepts, which is built based on skip-gram + regularization, but ontologies and every concept are mapped into a certain point of this space. For each model mapping to this medical concept space is also built from the vector representation which is generated by the model form a pipeline through the mapping function.

Then from this generic space the vectors for a particular patient are taken and transmitted to the final model, where they are aggregated into the unified patient model according to which the diagnosis is made and/or therapy is recommended, etc.

When all the required actions are executed, an administrator, doctor or other user adds (sends) patient records to be analyzed to the server.

An example of such record could be:

Man aged 60 complained about chest pain when walking, dyspnea. Description of ECG examination is attached to the health record. ECG has revealed sinus rhythm, QRS complex is normal, pronounced ST segment depression (1 mm) in V4-V6 leads. Therapy is not assigned. No differential diagnosis.

Then, the server preprocesses the data, selects key facts and transforms them into medical facts, for example:

```
<Chest pain>
<Dyspnea>
```

```
<QRS complex is normal>
<ST segment depression>
```

Thereafter, the server sends the obtained set of facts to the input of existing models and makes a diagnosis with the utmost probability corresponding with the submitted set of facts.

Then the server receives the results of model usage. The server outputs the following results of the model as an illustrative example: 75% corresponds to "angina" diagnosis, additional examinations are recommended: bicycle ergometry, daily ECG monitoring.

The results can be presented in the form of recommendations, selection of areas of interest in medical images, in the form of reports.

Analysis and prognosis of disease course could be as a result of model (models) applying.

In some embodiment's patient mortality could be predicted.

In some embodiments a list of queries enters the server. For example, query is one series of CT examination requiring processing by the server. The server executes a processing and, for example, it encircles (highlights) in red in CT slice a potential area of interest specified by the model, and all the obtained volumes of interest consisting of areas of interest are presented as a list. Area of interest is localized in the slice, volumes of interest are built as aggregation of several areas of interest into one.

In some embodiments cardiac insufficiency, liver diseases and other diseases (pathologies) could be predicted.

The experimental data on using this technical solution in one of its embodiments are given below.

Data from MIMIC-III (Medical Information Mart for Intensive Care III) - free access database, comprising anonymized health data associated with about 40 thousand of patients which stayed in the intensive care unit of Beth Israel Medical Center within the period 2001 - 2012, were used for the study.

MIMIC-III comprises information about demography, laboratory measurements, procedures, prescribed medicines, patient mortality and vital indications recorded during patient staying in the medical center.

For the purpose of different models comparison, the data were processed using the following approach.

First, from the database there was extracted the information on diagnosis (in the form of ICD9 codes, which had been used in the original database), prescribed medicines (in the form of NDC identifiers, or, if they were unavailable, in the form of medicine text description) and assigned procedures (in the form of procedure ICD9 codes) for each of the patients with reference to a particular appointment, arranged by appointment date.

As a result, the obtained dataset contained a large quantity of patients with brief but less informative history of visits to the medical center. It is possible to use the information about such patients to train weight matrix to establish relationships between the diagnoses. However, when training the models such information will not be useful: it is not possible to recognize an event occurred during the next visit to a doctor for patients visited the clinic only once.

Therefore, for preparation of the weight matrix all the patient information was used, and for model training the data were additionally processed: patient health record was scrolled by a sliding window 1 year long and all visits to the clinic recorded within this year were considered as an independent set of features, while the sliding windows with less than 2 visits were excluded from the consideration.

From each sequence of patient visits to the medical center within a year all appointments, except for the latest one, were extracted and used together with the relative information to extract the features transmitted to the input of specific models.

Then, all such sequences were divided into the training and testing datasets at a ratio of 4 to 1.

Additional processing can depend on what the specific model input receives.

MIMIC-III database is designed so that several diagnoses, medicines and procedures can be associated with every patient visit to the medical center, and their order inside the appointment is not uniquely defined. Therefore, if the model receives an ordered sequence of events and does not take the appointment time into consideration, then, when training this model, diagnoses, medicines and procedures inside one appointment are rearranged in random manner, and then, "appointments" are united into a sequence.

Since such sequence of events will have different length for different patients, and long-ago events will make less contribution to prediction of diagnoses, in some embodiments the models are learned from the latest N events (in case of less events in the patient health record they are added with zeros). The whole sequence of visits for each 1 year long window is considered for the model only.

Pretraining of the weight matrix

When building some classifiers, there were used so called embedding matrices to obtain MCV-vector or medical concept vector - contracted representation of the health record which is a finite length vector, which elements are real numbers.

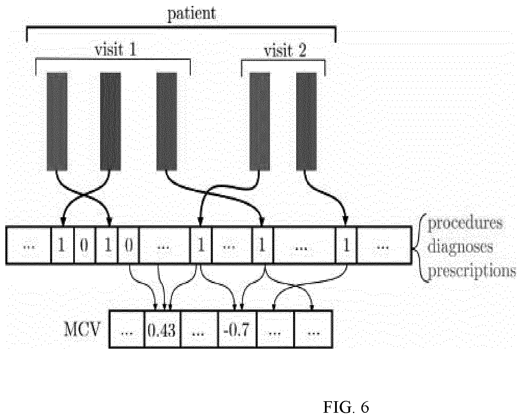

The scheme of obtaining the medical concept vectors for a patient is shown in Fig. 6.

Time-ordered events in the health record were considered for its building. If an event of a certain type occurred, we put " 1" into the vector position corresponding to the event. Thus, we obtain a vector of high dimension consisting of 1 - in those positions which correspond to the events occurred in the health record, and 0 - in the positions not occurred in the health record.

In some models this general scheme is modified: for example, events rearranged in random manner within one appointment (and one time mark) are considered, fixed amount of the latest events is recorded into the event vector, or events corresponding to one appointment are additionally multiplied by the weight which is defined by how long ago the event occurred.

Such sparse representation of the health record requires much memory, model training based on it takes much time. In order to reduce time and compact data the medical concept vector for a patient is drawn on the basis of the sparse representation of the health record.

For the purpose of obtaining the contracted representation at its simplest the so-called embedding-matrix is used, by which the sparse vector of the health record is multiplied. Several matrices have been considered:

Word2Vec: as a matrix we took a coefficient matrix obtained on the basis of the analysis of secondary diagnoses, directions and medicines, which were present within one appointment. For training we used word2vec model with skipgram mechanism so as to obtain the medical concept vector of a certain length for any diagnosis, medical procedure or prescribed medicine (corresponding to the embedding-matrix column).

This weight matrix was learned from the health record comprising codes of diagnoses, symptoms, prescribed procedures and prescribed medicines to extract more information about relations between diagnoses.

Ontology embedding: Ontology information was used, namely, the codes located in higher-level nodes of the diagnosis tree, expressed in terms of ICD9 codes, were used to obtain contracted representations of events.

Embedding with ICD9 tree: to obtain the contracted representation it is possible to use nonstandard regularization function, which maximizes the distance to far objects and minimizes the distance to near objects in the tree (and at the same time corrects the vectors for parent nodes in ICD9-code tree). As opposed to the existing approaches where the diagnosis weights and their parents in the tree are trained for a specific problem, the weight matrix is pretrained and being already trained it is used for model training.

For each of the set problems several classifiers could be considered:

Basic 1-hot encoding: this model was built on the basis of logistic regression to which input the array of 0 и 1 - sparse vector representation of the health record, was transmitted. Only diseases were considered.

TFI-DF encoding: this model was built on the basis of logistic regression to which input the array, which slots were associated with patient diseases, was transmitted. It was built analogously to the previous model except for the fact that the number of disease occurrence in the health record was taken into consideration, and then, the input features were processed by TF-IDF algorithm to associate larger weight with diagnoses of rare occurrence in general, but of frequent occurrence in a particular patient.

The following several models used similar neural network architecture for classification, but different weight matrices.

Word2Vec embeddings: the model used weight matrix to obtain the contracted representation of the health record in the form of the patient vector. Skip-gram-based Word2vec matrix was used as a weight matrix. The obtained contracted representations were used as features for logistic regression.

Word2Vec embedding+attention: Word2Vec weight matrix was used in the model to obtain the contracted representation of the patient vector inside the model. Besides, the neural network architecture with attention mechanism was used.

Embedding with ICD9 tree: model with embedding matrix, built on the basis of ICD9-code tree. Contracted representations of patients, obtained by multiplying the matrix by patient vectors, were used for building the model based on logistic regression.

Embedding with ICD9 tree+attention: model with embedding matrix, built on the basis of ICD9-code tree, in which the neural network architecture with attention mechanism was used.

Embedding with ICD9 tree+attention+tfidf: model which differs from the previous one in that the value, which the TFI-DF encoding model returned for the specified patient, was additionally transmitted to its input.

Choi embedding+attention: model with embedding matrix, built on the basis of vector contracted representations, considered in Choi et all "GRAM: Graph-based Attention Model for Healthcare Representation Learning", using the attention mechanism.

Time-based model: Method of drawing the patient mcv vectors is reproduced.

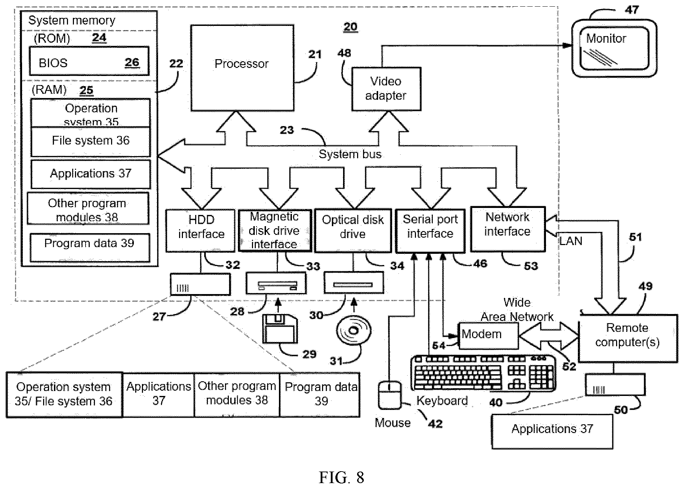

Fig. 8 illustrates the example of the general purpose computer system in which the current technical solution can be implemented, and which comprises a multipurpose computing unit in the form of a computer **20** or server comprising a processor **21,** system memory **22** and system bus **23,** which links different system components, including the system memory with the processor **21.**

The system bus **23** can be of any of different bus structure types including a memory bus or memory controller, peripheral bus and local bus using any of multiple bus architectures. The system memory includes read-only memory (ROM) **24** and random-access memory (RAM) **25.** ROM **24** stores basic input/output system **26** (BIOS), consisting of the programs which serve to exchange information between elements inside the computer **20,** for example, during start-up.

The computer **20** can also include hard disk drive **27** for reading from and writing to hard disk, magnetic disk drive **28** for reading from and writing to the removable disk **29,** and optical disk drive **30** for reading from and writing to removable optical disk **31** such as compact disk, digital video disk and other optical means. Hard disk drive**27**, magnetic disk drive **28** and optical disk drive **30** are connected to the system bus **23** by means of, respectively, hard disk drive interface **32,** magnetic disk drive interface **33** and optical disk drive interface**34**. Disk drives and their corresponding means readable by the computer ensure nonvolatile storage of instructions, data structures, program modules and other computer-readable data for the computer **20.**

Though typical configuration described herein uses hard disk, removable magnetic disk **29** and removable optical disk **31,** it is obvious to a person skilled in the art that in typical operation environment there could be used other types of computer-readable means which can store data accessible from the computer, such as magnetic cassettes, flash memory drives, digital video disks, Bernoulli cartridges, random access memories (RAM), read-only memories (ROM), etc.

Different program modules, including operating system **35,** can be saved to hard disk, magnetic disk **29,** optical disk **31,** ROM **24** or RAM **25.** The computer **20** comprises the file system **36,** linked to the operating system 35 or integrated into it, one or several software applications **37,** other program modules **38** and program data **39.** User can input commands and information to the computer **20** by means of such input devices as keyboard **40** and pointing device **42.** Other input devices (not shown) may include microphone, joystick, game pad, satellite antenna, scanner or any other.

These and other devices are commonly connected to the processor **21** by a serial port interface **46** which is linked to the system bus, but they can be connected by means of other interfaces such as parallel port, game port or universal serial bus (USB). Monitor **47** or other visual display unit is also connected to the system bus **23** by means of interface, e.g. video adapter **48.** In addition to monitor **47** personal computers normally comprise other peripheral output devices (not shown) such as speakers and printers.

Computer **20** can work in a network neighbourhood by means of logical connections to one or several remote computers **49.** Remote computer (or computers) **49** could be the other computer, server, router, networked PC, peer-to-peer device or other node of the single network and also normally comprises the majority or all the above elements with respect to the computer **20,** though storage device **50** is shown only. Logical connections include Local Area Network (LAN) **51** and Wide Area Network (WAN) **52.** Such network neighbourhoods are commonly used in offices, intranets, Internet.

The computer **20** used in LAN neighbourhood is connected to the local network **51** by network interface or adapter **53.** The computer **20** used in WAN neighbourhood normally uses a modem **54** or other devices for communication with global computer network **52** such as Internet.

Modem **54,** which can be internal or external, is connected to the system bus **23** by serial port interface **46.** In the network neighbourhood the program modules or their parts described with respect to the computer **20** can be stored in the remote storage device. It is necessary to consider that the illustrated network connections are typical and other means could be used for communication between computers.

One final comment is that the data given in the description are the examples which do not limit the scope of this technical solution defined by the claims. It is obvious to a person skilled in the art that there could exist other embodiments of this technical solution compliant with the essence and scope of this technical solution.

Literature:

1. https://hackernoon.com/attention-mechanism-in-neural-network-30aaf5e39512
2. https://medium.com/@Synced/a-brief-overview-of-attention-mechanism-13c578ba9129
3. "Medical Concept Representation Learning from Electronic Health Records and its Application on Heart Failure Prediction", Edward Choi, Andy Schuetz, Walter F. Stewart, Jimeng Sun, 11/02/2016.
4. "Graph-based attention model for healthcare representation learning", Edward Choi, Mohammad Taha Bahadori, Le Song, Walter F. Stewart, Jimeng Sun, 2017.

**Claims**

**1.** A method for supporting a medical decision using patient representation mathematical models performed on a server, comprising the following steps:

• forming a training dataset comprising electronic health records of patients grouped by each patient;
• performing a preliminary processing of data contained in the electronic health records selected from the training dataset;
• transforming the processed data into a sequence of medical facts per every patient using medical ontologies;
• performing automatic layout of the obtained sequence of medical facts per every patient using diagnoses or other facts of interest extracted from the health records;
• performing training of primary representations individually for each of modalities;
• performing training of joint representations;
• performing training of final models and aggregation parameters;
• obtaining a health record of a patient that is not included into the training dataset;
• performing the preliminary processing of data contained in the obtained health record of the patient;
• transforming the preliminarily processed data into a sequence of medical facts using medical ontologies;
• submitting the obtained sequence of medical facts to an input of the final models;
• making a diagnosis and also making an analysis and prognosis of a disease course for the patient that correspond to the obtained sequence of medical facts with greatest probability.

2. The method according to claim 1, in which electronic health records comprise at least the following data: patient's condition, methods of patient's treatment, means used to treat a patient, test results.

3. A system for supporting a medical decision using patient representation mathematical models, comprising at least one processor, a random-access memory, a storage device containing instructions downloaded into the random-access memory and executed by the at least one processor, the instructions comprise the following steps:

• forming a training dataset comprising electronic health records of patients grouped by each patient;
• performing a preliminary processing of data contained in the electronic health records selected from the training dataset;
• transforming the processed data into a sequence of medical facts per every patient using medical ontologies;
• performing automatic layout of the obtained sequence of medical facts per every patient using diagnoses or other facts of interest extracted from the health records;
• performing training of primary representations individually for each of modalities;
• performing training of joint representations;
• performing training of final models and aggregation parameters;
• obtaining a health record of a patient that is not included into the training dataset;
• performing the preliminary processing of data contained in the obtained health record of the patient;
• transforming the preliminarily processed data into a sequence of medical facts using medical ontologies;
• submitting the obtained sequence of medical facts to an input of the final models;
• making a diagnosis and also making an analysis and prognosis of a disease course for the patient that correspond to the obtained sequence of medical facts with greatest probability.

FIG. 1

Beginning

210 — forming a training dataset

220 — preliminary processing of data contained in the health record

230 — transforming the processed data into a sequence of medical facts per every patient using medical ontologies

240 — making automatic layout of the obtained sequence of medical facts per every patient using diagnoses or other facts of interest extracted from the health record

250 — training of primary representations individually for each of modalities

260 — training of joint representations

270 — training of final models and aggregation parameters

End

FIG. 2

FIG. 3

FIG. 4

FIG. 5

FIG. 6

Biomedical images → Preprocessing → Neural network model → Vector representation → Model to MCVS → Medical concept vector space

Medical terms database → Vectorization by means of Skip-gram → Model to MCVS → CT, X-ray, Fever, ECG

ECG → Preprocessing → Neural network model → Vector representation → Model to MCVS

FIG. 7

FIG. 8

FIG. 9A

FIG. 9B

EP 3 734 604 A1

**Training of joint representations**

Patient records on timeline · acute tonsillitis · azithromycin · acute rheumatic fever

fever · pharyngalgia · induration of cervical lymph nodes · ECG

Predicted neighbouring medical concepts for "induration of cervical lymph nodes" concept: fever, pharyngalgia, acute tonsillitis, azithromycin, induration of cervical lymph nodes

Predicted neighbouring medical concepts for "acute rheumatic fever" concept: ECG*, acute tonsillitis, azithromycin, acute rheumatic fever

*ECG - vector generated from native signal and processed by neural network from primary representations

FIG. 10A

**Neural network training**

Vector of medical concepts describing patient state → Input signal vector → Hidden layer → Hidden layer → Output layer → Norm/Pathology

FIG. 10B

## INTERNATIONAL SEARCH REPORT

| | International application No. |
|---|---|
| | PCT/RU 2017/000819 |

**A. CLASSIFICATION OF SUBJECT MATTER**

G16H 10/60 (2018.01); G16H 50/00 (2018.01)

According to International Patent Classification (IPC) or to both national classification and IPC

**B. FIELDS SEARCHED**

Minimum documentation searched (classification system followed by classification symbols)

G06F 3/00, 3/01, 3/048, 17/00, 17/30, 17/40, 19/00, G06N 5/00, 5/02, G16H 10/00, 10/60, 50/00-50/80

Documentation searched other than minimum documentation to the extent that such documents are included in the fields searched

Electronic data base consulted during the international search (name of data base and, where practicable, search terms used)

DWPI, Espacenet, K-PION, KIPO, PAJ, PatSearch (RUPTO internal), USPTO, WIPO

**C. DOCUMENTS CONSIDERED TO BE RELEVANT**

| Category* | Citation of document, with indication, where appropriate, of the relevant passages | Relevant to claim No. |
|---|---|---|
| X | US 2016/0364537 A1 (DASCENA) 15.12.2016, the abstract, paragraphs [0006], [0007], [0036], [0038], [0040], [0041], [0043]-[0045], [0050], [0051], [0057], [0084], [0087], [0105], [0113] | 1-3 |
| A | US 2017/0053064 A1 (SEMANTICMD, INC.) 23.02.2017 | 1-3 |
| A | US 2016/0378919 A1 (THE JOHNS HOPKINS UNIVERSITY) 29.12.2016 | 1-3 |
| A | RU 2515587 C1 (FEDERALNOE GOSUDARSTVENNOE BJUDZHETNOE UCHREZHDENIE NAUKI INSTITUT PROBLEM UPRAVLENIYA IM. V.A. TRAPEZNIKOVA ROSSIISKOI AKADEMII NAUK) 10.05.2014 | 1-3 |
| A | WO 2015/023674 A1 (CERNER INNOVATION, INC.) 19.02.2015 | 1-3 |

☐ Further documents are listed in the continuation of Box C.  ☐ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 16 August 2018 (16.08.2018) | 23 August 2018 (23.08.2018) |

| Name and mailing address of the ISA/ RU | Authorized officer |
|---|---|
| Facsimile No. | Telephone No. |

Form PCT/ISA/210 (second sheet) (July 1998)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **EDWARD CHOI ; ANDY SCHUETZ ; WALTER F. STEWART ; JIMENG SUN.** *Medical Concept Representation Learning from Electronic Health Records and its Application on Heart Failure Prediction,* 11 February 2016

- **EDWARD CHOI ; MOHAMMAD TAHA BAHADORI ; LE SONG ; WALTER F. STEWART ; JIMENG SUN.** *Graph-based attention model for healthcare representation learning,* 2017